(19) Europäisches Patentamt
European Patent Office
Office européen des brevets

(11) Publication number: **0 106 379**
**B1**

(12) # EUROPEAN PATENT SPECIFICATION

(45) Date of publication of patent specification: **13.08.86**

(21) Application number: **83201237.1**

(22) Date of filing: **29.08.83**

(51) Int. Cl.$^4$: **C 07 C 51/14,** C 07 C 53/122, C 07 C 51/56, C 07 C 67/38, C 07 C 69/24, C 07 C 69/612

(54) **Process for the carbonylation of olefinically unsaturated compounds with a palladium catalyst.**

(30) Priority: **30.09.82 NL 8203800**
**23.12.82 NL 8204963**

(43) Date of publication of application:
**25.04.84 Bulletin 84/17**

(45) Publication of the grant of the patent:
**13.08.86 Bulletin 86/33**

(84) Designated Contracting States:
**BE DE FR GB IT NL**

(56) References cited:
**US-A-3 501 518**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

**EP 0 106 379 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for the carbonylation of olefinically unsaturated compounds.

It is known that olefins may be carbonylated in the presence of water, alcohols or carboxylic acids to yield carboxylic acids, esters or carboxylic acid anhydrides, respectively. However, the known processes have various drawbacks which render them rather unattractive for use on a technical scale.

US patent specification 3,168,553 discloses a process in which olefins are carbonylated in the presence of a complex comprising a trialkylphosphine together with cobalt, ruthenium, rhodium or iridium. However, this process requires the use of high pressures, and its selectivity towards the desired product is often unsatisfactory. For instance, the carbonylation of ethylene in the presence of ethanol and $Co_2(CO)_8$ as the catalyst leads to the formation not only of ethyl propionate, but also of large quantities of by-products, such as diethyl ketone and acetaldehyde.

As appears from US patent specification 3,917,677, relatively high yields of ester can be obtained by using halogen-free rhodium catalysts in the carbonylation of olefins in the presence of alcohols and tertiary organophosphorus compounds. However, rhodium catalysts are costly, and cannot simply be replaced by cheaper palladium catalysts in the process according to US patent specification 3,917,677. Example 5 of this patent specification shows that no methyl propionate is formed when in the carbonylation of ethylene in the presence of methanol, sodium acetate and tributylphosphine at a temperature of 175°C and a pressure of 50 bar the palladium catalyst Pd $[(C_6H_5)_3P]_2Cl_2$ or $(CH_3COO)_2Pd$ is used instead of a rhodium catalyst.

A process for the preparation of carboxylic acids or esters by reacting an olefinically unsaturated compound with carbon monoxide in the presence of water or an alcohol, respectively, and a palladium catalyst has been disclosed in US patent specification 3,437,676. The catalyst has the formula $L_mPdX_n$, wherein L may be, inter alia, an organic phosphine, wherein X represents a chloride, bromide, sulphate, phosphate, acetate, propionate, nitrate or borate radical, and m is 1—4 and n is 1 or 2. The reaction is preferably carried out in the presence of an acid, such as sulphuric acid, phosphoric acid, boric acid or a carboxylic acid. Preference is given to the use of hydrochloric acid, because lower reaction temperatures may then be used. However, the pressures applied are very high; in all the examples concerning the carbonylation of ethylene or propylene the pressure is 709 bar. Moreover, reaction rates are low in spite of the presence of an acid. Lines 1 and 2 of column 14 show that for the carbonylation of ethylene in the presence of ethanol and reaction time usually amounts to 10—24 hours.

In Example 8 of DAS 2,263,442 ethylene is carbonylated at a pressure of about 50 bar in the presence of propionic acid, $PdCl_2$ $[(C_2H_5)_3P]_2$ and pentafluorothiophenol as the promoter. It is true that this process yields propionic anhydride, but it does so at a rate of less than 10 g anhydride per g Pd per hour. In this specification, too, there is a marked preference for the use of the costly iridium or rhodium catalysts.

From German Offenlegungsschrift 2,410,246 a process is known for the preparation of carboxylic acids, esters or carboxylic acid anhydrides by carbonylation of olefinically unsaturated compounds in the presence of a hydroxy compound, a zero valent triorganophosphine palladium or platinum complex and at least 10 mol, preferably 20—110 mol, of a triorganophosphine per mol palladium or platinum complex. In this process, at the reaction temperatures used in the examples concerning the conversion of ethylene or propylene, the total pressure amounts to more than 100 bar and long reaction times, of over 10 hours, are usually required.

European patent application 55875 discloses a process in which olefins are carbonylated in the presence of a halide-free palladium catalyst and water, an alcohol and/or a carboxylic acid, preferably at a pressure of 30—61 bar gauge. This process is carried out in the presence of less than 10 mol of a triorganophosphine having at least one aliphatic carbon atom bonded to the phosphorus atom per mol of palladium. Although it is stated that by the addition of water and/or a carboxylic acid the reaction rate can be increased, it remains rather low. In all the examples the reaction time used is 15 hours; the highest reaction rates achieved in Examples 6 and 7 in the presence of acetic acid or water are 11 and 12 g ester per g Pd per hour, respectively.

A process for the carbonylation of propylene in the presence of water or an alkanol and a palladium catalyst, 4—122 mole of an ortho-substituted triarylphosphine and, optionally, a promoter has been disclosed in European patent application 43382. Hydrohalogenic acids, particularly HCl, are the preferred promoters, although some experiments are also described using trifluoroacetic acid and phosphoric acid as promoters. The lowest pressure used in the examples is about 100 bar and the shortest reaction time 2 hours. Yields are not mentioned.

It has now surprisingly been found that in the carbonylation of olefinically unsaturated compounds the reaction rate can be very much enhanced by carrying out the reaction in the presence of an acid defined more closely hereinafter and at least 5 mol of a triarylphosphine per gram atom palladium.

The invention therefore relates to a process for the carbonylation of an olefinically unsaturated compound with carbon monoxide in the presence of water, an alcohol and/or a carboxylic acid, a palladium catalyst, at least 5 mol of a phosphine $PR^1R^2R^3$ in which $R^1$, $R^2$ and $R^3$ each represent an optionally substituted aryl group, per gram atom of palladium, and an acid as a promoter, characterized in that an acid with a $pK_a<2$ (at 18°C in aqueous solution), except hydrohalogenic and carboxylic acids, is used.

The acids used as promoters in the process according to the invention preferably have a

non-co-ordinating anion, by which is meant that little or no co-valent interaction takes place between the palladium and the anion. Typical examples of such anions are $PF_6^-$, $SbF_6^-$, $BF_4^-$ and $ClO_4^-$.

Acids preferably used are, for instance, sulphonic acids and those acids that can be formed, possibly in situ, by interaction of a Lewis acid such as, for example, $BF_3$, $AsF_5$, $SbF_5$, $PF_5$, $TaF_5$ or $NbF_5$ with a Broensted acid such as, for example, a hydrohalogenic acid, in particular HF, fluorosulphonic, phosphoric acid or sulphuric acid. Specific examples of the last-named type of acids are fluorosilicic acid, $HBF_4$, $HPF_6$ and $HSbF_6$. Typical sulphonic acids that can be used are fluorosulphonic acid, chlorosulphonic acid and the sulphonic acids specified hereinafter.

A preferred group of acids has the general formula

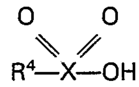

I

wherein X represents sulphur or chlorine and, if X is chlorine, $R^4$ represents oxygen and if X is sulphur, $R^4$ represents an OH group or an optionally substituted hydrocarbon group.

When the afore-mentioned acids are used in the process according to the invention, the anions of the acids can be regarded as non-co-ordinating.

The carbonylation of the olefinically unsaturated compounds should be carried out in the presence both of an acid as hereinbefore defined and of at least 5 mol of the phosphine mentioned. Further it is important that the catalyst be a palladium catalyst. It was found, for instance in the carbonylation of ethylene with carbon monoxide in the presence of methanol, palladium acetate and triphenylphosphine, that not unless a large excess of the phosphine (at least 5 mol per gram atom of palladium) is present as well, does the addition of for instance p-toluenesulphonic acid bring about a considerable increase in the reaction rate. It was further found that it is essential that the phosphine comprise three aryl groups bonded to the phosphorus atom. When using an excess of, for instance, tributylphosphine, ethyldiphenylphosphine or phenyldiethylphosphine, the addition of the acid produces practically no enhancement of the reaction rate. And also the addition of, for instance, p-toluene-sulphonic acid to a rhodium catalyst instead of a palladium catalyst, even in the presence of more than 5 mol triarylphosphine per gram atom Rh, will not produce the desired great enhancement of the carbonylation rate. Nor is this result achieved when, in the presence of excess triarylphosphine, one of the acids used in European patent application 43382, notably acetic acid, trifluoroacetic acid, hydrochloric acid or phosphoric acid, is added to a palladium catalyst.

By using the process according to the invention very high reaction rates—of the order of 6000 g product per g palladium per hour—can be achieved in spite of the moderate reaction conditions used. Since these reaction rates are much higher than hitherto attained by known methods, the present invention is of great importance.

In the acids having the general formula I, the optionally substituted hydrocarbon group, represented by $R^4$, is preferably an alkyl, aryl, aralkyl or alkaryl group with 1—30, in particular 1—14, carbon atoms. The hydrocarbon group may be substituted with for instance halogen atoms, in particular fluorine atoms. Examples of suitable acids of the general formula I are perchloric acid, sulphuric acid 2-hydroxypropane-2-sulphonic acid, p-toluenesulphonic acid and trifluoromethane sulphonic acid with the latter two acids being preferred. The acid of the general formula I may also be an ion exchange material which comprises sulphonic acid groups such as Amberlite 252 H. In this case the hydrocarbon group $R^4$ is a polymer hydrocarbon group substituted with sulphonic acid groups, for instance a polystyrene group.

The quantity of the acid with a $pK_a<2$ present in the reaction mixture is preferably 0.01—150, more particularly 0.1—100, and most preferably 1—50 equivalents per gram atom of palladium. The acid can optionally be formed in situ, for example of hydrolysis of an ester, such as for instance an alkyl ester of a sulphonic acid, or by reacting a ketone with $SO_2$ and water.

The olefinically unsaturated compound may be an unsubstituted or a substituted alkene or cycloalkene preferably having 2—30, and in particular 2—20, carbon atoms and preferably 1—3 double bonds. The alkene or cycloalkene may be substituted, for instance, with one or more halogen atoms or cyano, ester, alkoxy, hydroxy, carboxy or aryl groups. If the substituents are not inert under the reaction conditions, the carboxylation reaction may be accompanied with other reactions. For instance, the carbonylation of allyl alcohol is accompanied with esterification of the hydroxy group. Examples of suitable olefinic compounds are ethene, propene, butene-1, butene-2, isobutene, the isomeric pentenes, hexenes, octanes and dodecenes, cyclooctadiene-(1,5), cyclododecene, cyclododecatriene-(1,5,9), allyl alcohol, methyl acrylate, ethyl acrylate, methyl methacrylate, acrylonitrile, acrylamide, N,N-dimethyl acrylamide, viny chloride, allyl chloride, acrolein, oleic acid, methyl allyl ether and styrene.

The alcohols or carboxylic acids used in the process according to the invention may be aliphatic, cycloaliphatic or aromatic and may be substituted with one or more substituents, such as mentioned hereinbefore in connection with the olefinically unsaturated compounds to be used as starting material. The alcohol may therefore also be a phenol. The alcohols or carboxylic acids preferably contain not more than 20 carbon atoms. Examples of suitable alcohols or carboxylic acids are methanol, ethanol, propanol, isobutanol, tert.butanol, stearyl alcohol, benzyl alcohol, cyclohexanol, allyl alcohol, chlorocapryl alcohol,

ethylene glycol, propanediol-(1,2), butanediol-(1,4), glycerol, polyethylene glycol, hexanediol-(1,6), phenol, cresol, acetic acid, propionic acid, butyric acid, caproic acid, trimethylacetic acid, benzoic acid, caprylic acid, succinic acid, adipic acid and hydroxycaproic acid. Special preference is given to alkanols and carboxylic acids having 1—10 carbon atoms. If the alcohol or the carboxylic acid has more than one hydroxy group or carboxy group, different products may be formed, depending on the molar ratios existing between the reagents. For instance, depending on the quantity of olefinically unsaturated compound used, either a mono-ester or a diester may be produced from glycerol.

The products formed in the process according to the invention may be further reacted if desired. For instance, the carbonylation of an olefin, when conducted in the presence of water, yields a carboxylic acid which, by reaction with a further quantity of olefin, may form a carboxylic acid anhydride. When the carbonylation is carried out in the presence of an alcohol, it yields an ester which, when water is present as well, may hydrolyze to form an acid and an alcohol, each of which may again react with an olefin. When the carbonylation is carried out in the presence of a carboxylic acid, it yields an acid anhydride which, when water is present as well, may hydrolyze to form one or more carboxylic acids which in their turn may react with a further quantity of olefin.

Reaction of an alkanecarboxylic acid having n+1 carbon atoms with an olefin having n carbon atoms yields the symmetrical anhydride of the alkanecarboxylic acid having n+1 carbon atoms. This anhydride may optionally be hydrolyzed, half of the carboxylic acid formed may be collected as a product and the other half recycled to the carbonylation reactor. The process thus leads to the conversion of an olefin having n carbon atoms into a carboxylic acid having n+1 carbon atoms.

Both homogeneous and heterogeneous palladium catalysts may be used in the process according to the invention. However, homogeneous catalysts are preferred. Suitable homogeneous catalysts are the salts of palladium with, for instance, nitric acid, sulphuric acid or alkanecarboxylic acids having not more than 12 carbon atoms. Salts of hydrohalogenic acids may, in principle, be used as well, but they have the drawback that the halogen ion may have a corrosive effect. A catalyst used by preference is palladium acetate. Moreover, palladium complexes may be used, for instance palladium acetylacetonate, tetrakistriphenylphosphinepalladium, bis-tri-o-tolylphosphinepalladium acetate or bis-triphenylphosphinepalladium sulphate. Palladium on charcoal and palladium bonded to an ion exchanger—for instance an ion exchanger comprising sulphonic acid groups—are examples of suitable heterogeneous catalysts.

The quantity of palladium catalyst is not critical. Preference is given to the use of quantities in the range between $10^{-5}$ and $10^{-1}$ gram atom palladium per mol of olefinically unsaturated compound.

The substituted or unsubstituted aryl groups $R^1$, $R^2$ and $R^3$ of the phosphine $PR^1R^2R^3$ preferably contain not more than 18, in particular 6—14, carbon atoms. Examples of suitable $R^1$, $R^2$ and $R^3$ groups are the naphthyl group and in particular the phenyl group. Suitable substituents are halogen atoms and alkyl, aryl, alkoxy, carboxy, carbalkoxy, acyl, trihalogenmethyl, cyano, dialkylamino, sulphonylalkyl and alkanoyloxy groups.

Examples of suitable phosphines are tri-p-tolylphospine, tri-p-methoxyphenylphosphine, o-diphenylphosphinobenzoic acid and in particular triphenylphosphine. The phosphine is used in a quantity of at least 5 mol, preferably 10—150 mol per gram atom of palladium. If the palladium catalyst already contains phosphine, this should be taken into account when calculating the amount of phosphine to be used.

In the process according to the invention the carbon monoxide may be used pure or diluted with an inert gas, such as nitrogen, noble gases or carbon dioxide. Generally the presence of more than 10%v of hydrogen is undesirable, since under the reaction conditions it may cause hydrogenation of the olefinic compound. Generally preference is given to the use of carbon monoxide or a carbon monoxide-containing gas which contains less than 5%v of hydrogen.

The carbonylation according to the invention is preferably carried out at a temperature in the range between 50 and 200°C, in particular between 75 and 150°C. The overall pressure preferably lies between 1 and 100, in particular 20 and 75, bar gauge.

The molar ratio of the olefinically unsaturated compound to water, alcohol or carboxylic acid is not critical. The molar ratio between hydroxy groups and olefinic double bonds may lie for instance between 0.1:1 and 10:1. When using a mono-olefin and either water, a monohydric alcohol or a monobasic acid, preference is usually given to the use of an excess of the hydroxy compound mentioned. However, when using a polyhydric alcohol or a polybasic acid to prepare a polyester or a polyanhydride, it will generally be necessary to use an excess of olefinic compound.

The process according to the invention may be carried out batchwise, continuously or semi-continuously. Generally there is no need for the use of a solvent since usually there will be an excess of one of the reactants—for instance the alcohol—which may serve as a solvent as well. If required, however, a solvent may be used, for instance dimethyl sulphoxide, diisopropyl sulphone, sulfolane, acetone, chloroform, methyl isobutyl ketone, diglym (dimethyl ether of diethylene glycol) or diisopropyl ether. The primary reaction product of the carbonylation reaction may also be used as a solvent.

Example 1

A 250-ml magnetically stirred Hastelloy C autoclave ("Hastelloy" is a trade mark) was charged with 50 ml methanol, 0.1 mmol $(CH_3COO)_2Pd$ and the quantities given in Table A of triphenylphosphine and

p-toluenesulphonic acid or trifluoromethanesulphonic acid. The autoclave was flushed with carbon monoxide, filled with ethane at a pressure of 20 bar and carbon monoxide at a pressure of 30 bar, sealed and heated to a temperature of 135°C. After the reaction time mentioned in Table A the contents of the autoclave were analysed by gas-liquid chromatography.

Experiments 1 and 2 of Table A are comparative examples. Experiment 1 shows that despite the presence of a large excess of triphenylphosphine (30 mol per gram atom Pd), the rate at which methyl propionate is formed is relatively low in the absence of an acid. As shown in Experiment 2, this rate is equally low when in the presence of a sulphonic acid less than 5 mol of triphenylphosphine is used per gram atom of Pd. Experiments 3—8 show that according to the invention the presence both of a sulphonic acid and of a relatively large excess of phosphine leads to a massive increase of the reaction rate.

In Experiments 4, 5, 6 and 7 the overall pressure at the end of the reaction was below 10 bar, which shows that very high conversions are obtained at exceptionally low pressures.

Experiment 5 was repeated using 0.1 mmol acetylacetonatodicarbonylrhodium instead of 0.1 mmol $(CH_3COO)_2Pd$ and a temperature of 150°C instead of 135°C. The yield of methyl propionate after a reaction time of 5 hours was 3.5 g. Thus, the reaction rate was only 68 g ester per g RH per hour. This comparative example shows that in the process according to the invention the presence of a palladium catalyst is of importance.

TABLE A

| Exp. No. | Triphenyl-phosphine (mmol) | Acid (mmol) | Reaction time (h) | Methyl propionate (g) | Reaction rate (g ester/g Pd/h) |
|---|---|---|---|---|---|
| 1 | 3 | — | 5 | 0.5 | 10 |
| 2 | 0.3 | pTS*(2) | 5 | 5 | 100 |
| 3 | 0.5 | pTS (2) | 1 | 8 | 800 |
| 4 | 1 | pTS (2) | 0.25 | 17 | 6800 |
| 5 | 3 | pTS (2) | 0.25 | 16 | 6400 |
| 6 | 3 | pTS (4) | 0.25 | 17 | 6800 |
| 7 | 3 | $CF_3SO_3H$ (2) | 0.25 | 15 | 6000 |
| 8 | 3 | pTS (0.5) | 0.25 | 7.75 | 3100 |

* pTS=p-toluenesulphonic acid.

Example 2

In the way described in Example 1 a number of experiments were carried out in which, instead of ethene, propene was used at a pressure of 8 bar. Moreover, in some of these experiments the triphenylphosphine was replaced by tri-n-butylphoshine, ethyldiphenylphosphine, diethylphenylphosphine or tri-(p-methoxyphenyl)phosphine. In addition, experiments were carried out using HCl, phosphoric acid, acetic acid, trifluoroacetic acid or benzenephosphoric acid instead of p-toluenesulphonic acid or trifluoromethanesulphonic acid. The results are tabulated in Table B.

TABLE B

| Exp. No. | Phosphine (mmol) | Acid (mmol) | Reaction time (h) | Methyl butyrate + methyl isobutyrate (g) | Reaction rate (g ester/g Pd/h) | Unbranched ester (%w) |
|---|---|---|---|---|---|---|
| 1 | Triphenylphosphine (3) | — | 5 | traces | — | — |
| 2 | " | pTS* (2) | 1 | 14.7 | 1470 | 70 |
| 3 | " | pTS (2) $+H_2O$ (10 mmol) | 1 | 17 | 1700 | 70 |
| 4 | " | $HClO_4$** (2) | 0.5 | 16 | 3200 | 72 |
| 5 | " | $H_2SO_4$ (2) | 0.5 | 17 | 3400 | 73 |
| 6 | " | $CF_3SO_3H$ (2) | 0.5 | 16 | 3200 | 70 |
| 7 | Tri-p-methoxyphenyl-phosphine (3) | pTS (2) | 1 | 15 | 1500 | 74 |
| 8 | Triphenylphosphine (3) | HCl*** (2) | 5 | 4.8 | 96 | 56 |
| 9 | " | $H_3PO_4$**** (2) | 5 | 5.1 | 102 | 72 |
| 10 | " | $CF_3COOH$ (2) | 5 | 2.6 | 52 | 73 |
| 11 | " | $C_6H_5PO(OH)_2$ (2) | 2.5 | 2.4 | 96 | 70 |
| 12 | " | $CH_3COOH$ (150) | 5 | traces | — | — |
| 13 | Tri-n-butylphosphine (3) | pTS (2) | 5 | 0.4 | 8 | 68 |
| 14 | Tri-n-butylphosphine (1.5) | pTS (2) | 5 | 0.5 | 10 | 50 |
| 15 | Ethyldiphenylphosphine (3) | pTS (2) | 5 | 1.5 | 30 | 68 |
| 16 | Phenyldiethylphosphine (3) | pTS (2) | 5 | 0.5 | 10 | 60 |

* pTS=p-toluenesulphonic acid
** as a 70%w aqueous solution
*** 0.2 ml 37%w HCl
**** as a 85%w $H_3PO_4$

Table B shows that the presence of three aryl groups in the phosphine is needed to attain a marked increased of the reaction rate. It further shows that the promoter activity of hydrochloric acid, phosphoric acid, acetic acid, trifluoroacetic acid and phenylphosphonic acid is much smaller than that of the acids used according to the invention. Comparison of Examples 3 and 8 shows that it is unlikely that the presence of water is responsible for the lower promoter activity of HCl. In Experiments 2—7 the propene conversion was virtually 100%.

Example 3

The experiment of Example 1 was repeated using 8 g dodecene-1 instead of ethene. In addition to 0.1 mmol $(CH_3COO)_2Pd$ were present 3 mmol triphenylphosphine and 2 mmol p-toluenesulphonic acid. After a reaction time of 30 minutes 90% of the olefin had been converted. The ester yield was 9.6 g; the unbranched ester content was 78% w. The reaction rate was 1900 g/g Pd/hour.

Example 4

The experiment of Example 3 was repeated using 10 g styrene instead of 8 g dodecene. After a reaction time of 5 hours the styrene conversion was virtually 100%. The yield of methylester of 2-phenylpropionic acid was 2.7 g and of methylester of 3-phenylpropionic acid 12.4 g.

Example 5

The experiment of Example 1 was repeated using 20 ml water instead of 50 ml methanol. The solvent present was 30 ml diglym (dimethyl ether of diethylene glycol). The reaction mixture further contained 0.1 mmol $(CH_3COO)_2Pd$, 3 mmol triphenylphosphine and 2 mmol p-toluenesulphonic acid. After a reaction time of 3 hours the yield of propionic acid was 13.1 g.

Example 6

The experiment of Example 1 was repeated using 50 ml acetic acid instead of 50 ml methanol. After a reaction time of 1 hour the yield of acetic anhydride was 8.4 g and of propionic acid 6.1 g. First the mixed anhydrides of acetic acid and propionic acid are formed, which react with the excess acetic acid present to form acetic anhydride and propionic acid.

Example 7

A 250-ml magnetically stirred Hastelloy C autoclave ("Hastelloy" is a trade mark) was charged with 50 ml methanol, 0.1 mmol $(CH_3COO)_2Pd$, 3 mmol triphenylphosphine and 1.5 mmol $HBF_4$ (as a 60%w aqueous solution). The autoclave was flushed with carbon monoxide, filled with propene at a pressure of 8 bar and carbon monoxide at a pressure of 30 bar, sealed and heated to a temperature of 120°C. After a reaction time of 2 hours the contents of the autoclave were analysed by gas-liquid chromatography. The aggregate yield of methyl butyrate and methyl isobutyrate amounted to 17 g with an unbranched ester content of 73%. The reaction rate was 850 g ester/g Pd/hour.

Example 8

The experiment of Example 7 was repeated using 15 ml methanol instead of 50 ml methanol, 4 mmol p-toluenesulphonic acid instead of 1.5 mmol $HBF_4$ and 3 mmol o-diphenylphosphinobenzoic acid instead of triphenylphosphine. As solvent, 35 ml of chloroform were present. After 5 hours at 135°C the aggregate yield of methyl butyrate and methyl isobutyrate amounted to 13.5 g with an unbranched ester content of 83%. The reaction rate was 270 g ester/g Pd/hour.

Example 9

A 250-ml magnetically stirred Hastelloy C autoclave ("Hastelloy" is a trade mark) was charged with 20 ml octene-1, 10 g phenol, 30 ml sulfolane and 0.1 mmol $(CH_3COO)_2Pd$, 20 mmol triphenylphosphine and 4 mmol p-toluenesulphonic acid. The autoclave was flushed with carbon monoxide, filled with carbon monoxide at a pressure of 50 bar, sealed and heated to a temperature of 135°C. After a reaction time of 5 hours the contents of the autoclave were analysed by gas-liquid chromatography. The total yield of phenylesters was 17.2 g with an unbranched ester content of 82%. The reaction rate was 320 g ester/g Pd/hour.

Example 10

In the way described in Example 1 an experiment was carried out with $H_2SiF_6$. The autoclave was charged with 50 ml methanol, 0.1 mmol $(CH_3COO)_2Pd$, 3 mmol triphenylphosphine and 1 mmol $H_2SiF_6$ being used in a 34%w aqueous solution. The autoclave was flushed with carbon monoxide, filled with propene at a pressure of 8 bar and carbon monoxide at a pressure of 30 bar, sealed and heated at a temperature of 120°C for 2 hours. 8.5 g of butyric esters were obtained, with an unbranched ester content of 70%. The reaction rate was 425 g ester/g Pd/hour.

**Claims**

1. A process for the carbonylation of an olefinically unsaturated compound with carbon monoxide in the presence of water, an alcohol and/or a carboxylic acid, a palladium catalyst, at least 5 mol of a phosphine $PR^1R^2R^3$ in which $R^1$, $R^2$ and $R^3$ each represent an optionally substituted aryl group, per gram atom of palladium, and an acid as a promoter, characterized in that an acid with a $pK_a<2$ (at 18°C in aqueous solution), except hydrohalogenic and carboxylic acids, is used.

2. A process as claimed in claim 1, characterized in that an acid with a non-co-ordinating anion is used as promoter.

3. A process as claimed in claim 1 or 2, characterized in that a sulphonic acid or an acid that can be formed by interaction of a Lewis acid with a Broensted acid is used as promoter.

4. A process as claimed in claim 1 or 2, characterized in that an acid having general formula

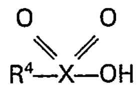

I

wherein X represents sulphur or chlorine and, if X is chlorine, $R^4$ represents oxygen and if X is sulphur, $R^4$ represents an OH group or an optionally substituted hydrocarbon group, is used as promoter.

5. A process as claimed in claim 4, characterized in that the optionally substituted hydrocarbon group represented by $R^4$ is an alkyl, aryl, aralkyl or alkaryl group having 1—30 carbon atoms.

6. A process as claimed in claim 4 or 5, characterized in that the acid is p-toluenesulphonic acid or trifluoromethanesulphonic acid.

7. A process as claimed in claims 1—6, characterized in that the acid is present in a quantity of 0.01—150, in particular 1—50, equivalents per gram atom of palladium.

8. A process as claimed in claims 1—7, characterized in that the olefinically unsaturated compound is an unsubstituted or substituted alkene or cycloalkene having 2—30 carbon atoms and 1—3 double bonds.

9. A process as claimed in claims 1—8, characterized in that the alcohol or the carboxylic acid has not more than 20 carbon atoms.

10. A process as claimed in claims 1—9, characterized in that the aryl groups represented by the groups $R^1$, $R^2$ and $R^3$ have 6—14 carbon atoms.

11. A process as claimed in claims 1—10, characterized in that the aryl groups represented by $R^1$, $R^2$ and $R^3$ are phenyl groups.

12. A process as claimed in claims 1—11, characterized in that 10—150 mol phosphine is used per gram atom palladium.

13. A process as claimed in claims 1—12, characterized in that the carbonylation is carried out at a temperature in the range between 50 and 200°C.

14. A process as claimed in claims 1—13, characterized in that the carbonylation is carried out at an overall pressure in the range between 1 and 100, in particular between 20 and 75, bar gauge.

**Patentansprüche**

1. Verfahren zur Carbonylierung einer olefinisch ungestättigten Verbindung mit Kohlenmonoxid in Gegenwart von Wasser, einem Alkohol und/oder einer Carbonsäure, einem Palladium-Katalysator, zumindest 5 mol eines Phosphins $PR^1R^2R^3$, worin $R^1$, $R^2$ und $R^3$ eine gegebenenfalls substituierte Arylgruppe ist, je g-Atom Palladium und einer Säure als Promotor, dadurch gekennzeichnet, daß man eine Säure mit einem Wert $pK_a<2$ (bei 18°C in wässriger Lösung) verwendet mit Ausnahme von Halogenwasserstoffsäuren und Carbonsäuren.

2. Verfahren nach Anspruch 1, dadurch gekennzeichnet, daß, man als Promoter eine Säure mit einem nicht-coordinativen Anion verwendet.

3. Verfahren nach Anspruch 1 oder 2, dadurch gekennzeichnet, daß man als Promoter eine Sulfonsäure oder eine Säure verwendet, die sich durch Umsetzung einer Lewis-Säure mit einer Broensted-Säure bildet.

4. Verfahren nach Anspruch 1 oder 2, dadurch gekennzeichnet, daß man eine Säure mit der allgemeinen Formel

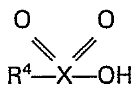

I

verwendet, worin X Schwefel oder Chlor ist und wenn X Chlor ist, $R^4$ Sauerstoff bedeutet, und wenn X Schwefel ist, $R^4$ eine OH-Gruppe oder eine gegebenenfalls substituierte Kohlenwasserstoffgruppe ist.

5. Verfahren nach Anspruch 4, dadurch gekennzeichnet, daß die gegebenenfalls substituierte Kohlenwasserstoffgruppe $R^4$ eine Alkyl; Aryl; Aralkyl-oder Alkarylgruppe mit 1 bis 30 Kohlenstoffatomen ist.

6. Verfahren nach Anspruch 4 oder 5, dadurch gekennzeichnet, daß man als Säure p-Toluolsulfonsäure oder Trifluormethansulfonsäure verwendet.

7. Verfahren nach Anspruch 1 bis 6, dadurch gekennzeichnet, daß man die Säure in einer Menge von 0,01 bis 150, insbesondere 1 bis 50 eq/g-Atom Palladium einsetzt.

8. Verfahren nach Anspruch 1 bis 7, dadurch gekennzeichnet, daß man als olefinisch ungesättigte Verbindung ein nicht-substituiertes oder substituiertes Alken oder Cycloalken mit 2 bis 30 Kohlenstoffatomen und 1 bis 3 Doppelbindungen verwendet.

9. Verfahren nach Anspruch 1 bis 8, dadurch gekennzeichnet, daß man einen Alkohol oder eine Carbonsäure mit nicht mehr als 20 Kohlenstoffatomen verwendet.

10. Verfahren nach Anspruch 1 bis 9, dadurch gekennzeichnet, daß die Arylgruppen der Substituenten $R^1$, $R^2$ und $R^3$ 6 bis 14 Kohlenstoffatome enthalten.

11. Verfahren nach Anspruch 1 bis 10, dadurch gekennnzeichnet, daß die Arylgruppen der Substituenten $R^1$, $R^2$ und $R^3$ Phenylgruppen sind.

12. Verfahren nach Anspruch 1 bis 11, dadurch gekennzeichnet, daß man 10 bis 150 mol Phosphin/g-Atom Palladium verwendet.

13. Verfahren nach Anspruch 1 bis 12, dadurch gekennzeichnet, daß man die Carbonylierung bie einer Temperatur zwischen 50 und 200°C durchführt.

14. Verfahren nach Anspruch 1 bis 13, dadurch gekennzeichnet, daß man die Carbonylierung bei einem Gesamtdruck zwischen 1 und 100, insbesondere zwischen 20 und 75 bar durchführt.

**Revendications**

1. Un procédé pour la carbonylation d'un composé oléfiniquement insaturé avec de l'oxyde de carbone en présence d'eau, d'un alcool et/ou d'un acide carboxylique, d'un catalyseur au palladium, d'au moins 5 moles d'une phosphine $PR^1R^2R^3$ où $R^1$, $R^2$ et $R^3$ représentent chacun un groupe aryle éventuellement substitué, par atome-gramme de palladium, et d'un acide comme promoteur, caractérisé en ce qu'on utilise un acide ayant un $pK_a$ inférieur à 2 (à 18°C en solution aqueuse), à l'exception des acides halogénhydriques et des acides carboxyliques.

2. Un procédé selon la revendication 1, caractérisé en ce qu'on utilise comme promoteur un acide ayant un anion non-coordinateur.

3. Un procédé selon la revendication 1 ou 2, caractérisé en ce qu'on utilise comme promoteur un acide sulfonique ou un acide qui peut être formé par interaction d'un acide de Lewis avec un acide de Broensted.

4. Un procédé selon la revendication 1 ou 2, caractérisé en ce qu'on utilise comme promoteur un acide ayant la formule générale

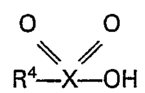

I

où X représente du soufre ou du chlore et, si X est du chlore, $R^4$ représente de l'oxygène et si X est du soufre, $R^4$ représente un groupe OH ou un groupe d'hydrocarbure éventuellement substitué.

5. Un procédé selon la revendication 4, caractérisé en ce que le groupe d'hydrocarbure éventuellement substitué représenté par $R^4$ est un groupe alcoyle, aryle, aralcoyle ou alcaryle ayant 1—30 atoms de carbone.

6. Un procédé selon la revendication 4 ou 5, caractérisé en ce que l'acide est de l'acide p-toluènesulfonique ou de l'acide trifluorométhanesulfonique.

7. Un procédé selon les revendications 1—4, caractérisé en ce que l'acide est présent à raison de 0,01—150, en particulier 1—50 équivalents par atome-gramme de palladium.

8. Un procédé selon les revendications 1—7, caractérisé en ce que le composé oléfiniquement insaturé est un alcène ou cycloalcène substitué ou non ayant 2—30 atomes de carbone et 1—3 doubles liaisons.

9. Un procédé selon les revendications 1—8, caractérisé en ce que l'alcool ou l'acide carboxylique n'a pas plus de 20 atomes de carbone.

10. Un procédé selon les revendications 1—9, caractérisé en ce que les groupes aryle représentés par les groupes $R^1$, $R^2$ et $R^3$ ont 6—14 atomes de carbone.

11. Un procédé selon les revendications 1—10, caractérisé en ce que les groupes aryle représentés par $R^1$, $R^2$ et $R^3$ sont des groupes phényle.

12. Un procédé selon les revendications 1—11, caractérisé en ce qu'on utilise 10—150 moles de phosphine par atome-gramme de palladium.

13. Un procédé selon les revendications 1—12, caractérisé en ce que la carbonylation est effectuée à une température comprise entre 50 et 200°C.

14. Un procédé selon les revendications 1—13, caractérisé en ce que la carbonylation est effectuée à une pression manométrique totale comprise entre 1 et 100, en particulier entre 20 et 75 bars.